# EUROPEAN PATENT SPECIFICATION

(11) **EP 0 164 818 B1**
(45) Date of publication and mention of the grant of the patent: **20.10.1993**
(21) Application number: 85301303.5
(22) Date of filing: 26.02.1985
(51) Int. Cl.: A61L 9/01

(54) **Process for counteracting pungency of ammoniacal substances**
Verfahren zur Bekämpfung starker Gerüche von Ammoniakverbindungen
Procédé pour combattre l'odeur irritante de substances ammoniacales

(30) Priority: 12.06.1984 US 619875
(43) Date of publication of application: 18.12.1985
(73) Proprietor: Nu Tech Ventures, Ltd., Denver Colorado 80216 (US)
(72) Inventor: Cox, James P., Lynden Washington 98264 (US); Cox, Kelly K., Lynden Washington 98264 (US)
(74) Representative: Everitt, Christopher James Wilders

(56) References cited:
- FR-A- 1 354 159
- FR-A- 1 485 781
- FR-A- 2 259 620
- FR-A- 2 416 016
- US-A- 2 905 591
- US-A- 3 775 334
- US-A- 3 787 566
- US-A- 4 007 262

## Description

This invention relates to counteracting malodors or pungency especially those produced by ammoniacal substances, that is, ammonia or ammonia-producing substances.

Ammonia or ammonia-producing substances occur in a wide variety of industrial or commercial processes or operations. In raising poultry, for example, either for meat or egg production, the poultry excrement and urine liberate ammonia or ammonia-producing substances such as amines. Such excrement may be utilized for or processed for use as fish feed, livestock feed or even chicken feed. Poultry excrement can be used for fertilizer.

Ammoniacal pungency also is produced in rendering residue resulting from butchering chickens such as poultry parts and feathers.

Further, ammoniacal pungency occurs in the rearing of livestock such as pigs and cattle in pens or feedlots caused by the urine and excrement of such livestock.

Ammoniacal pungency may result from air-carried ammoniacal substances such as in the air of poultry or livestock rendering plants. Ammoniacal substances may also be present in wash water used for cleansing such plants or equipment used in such plants.

Neckermann et al. U.S. patent No. 3,816,577, issued June 11, 1974, points out that deodorizer material for animal cages has consisted mainly of inert absorbent particulate material for absorbing animal waste, particularly urine, and proposes the use of water-soluble solids of cherry pits to react with animal waste, especially urine, to chemically tie up or otherwise remove odoriferous components of animal waste (column 5, line 18). In producing a deodorizing substance, the oil of the cherry pits down to preferably no more than 5 percent by weight is removed, the patent stating that such cherry pit oil is valuable in itself and does not significantly contribute to the effectiveness of the animal waste deodorizer (column 3, lines 30 to 37).

Roehm U.S. patent No. 3,459,852, issued August 5, 1969, points out at column 1, lines 56 and 57, that typical practice to remove sewage odors involves oxidation by aeration, possibly with an additional oxidizing agent, for example, sodium nitrate. Roehm utilizes alpha,beta aliphatically-unsaturated aldehydes and ketones, which he calls "sulfide-active" compounds, such as acrolein and 3-buten-2-one, for rendering the sulfide content of raw sewage essentially nonodorous. These preparations can also be used in scrubbing towers where sewage gases may be treated by exposing them to the chemicals, as stated at column 9, lines 62 to 65. A stabilizer, such as hydroquinone, is preferably used with the acrolein or other "sulfide-active" compounds, as stated at column 3, lines 15 to 18.

Goldstein U.S. patent No. 2,228,993, issued January 14, 1941, discloses the activation of benzyl alcohol (page 3, column 1, lines 53 to 57) with phenyl ethyl alcohol and myrrh resin, or with myrrh resin and oil of bergamot, or with myrrh resin and anisic aldehyde, as stated on page 3, column 2, lines 5 to 21, for the purpose of destroying odors, such as those produced by burning rubber, carbon disulfide and putrifying bodies (page 1, column 2, lines 14 and 15). The treating material may be diffused mechanically into the duct of an air-conditioning system such as of an industrial or air-conditioning plant or theater, as indicated at page 1, column 1, lines 3, 4 and 31 to 33. While the aldehyde principally proposed is anisic aldehyde (page 1, column 2, lines 37 and 41; page 3, column 2, lines 16, 21, 30 and 33), Goldstein points out that cinnamic aldehyde may be used instead of anisic aldehyde at page 2, column 2, lines 32 and 56, and page 3, column 2, line 31.

Grady U.S. patent No. 2,317,908, issued April 27, 1943, proposes to destroy odors arising from both inorganic and organic substances in zoos, hatcheries and stables, for example, by sprinkling dry deodorant composition or spraying wet composition on the floor. The deodorant composition is a mixture of marl and lime. The lime, whether slaked or unslaked, is asserted to produce an activating effect in the deodorant.

Peterson U.S. patent No. 3,706,663, issued December 19, 1972, states that a major problem associated with high-density type livestock operations is that of animal waste odors, in particular low molecular weight nitrogen-containing compounds. The patent discloses treating animal waste with sulfa drugs.

The Komakine U.S. patent No. 3,776,188, issued December 4, 1973, refers to noxious gases such as ammonia and hydrogen sulfide reeking in chicken houses (column 1, lines 23 and 24). This patent advocates dusting coarse powder of ferrous sulfate hepta-hydrate as a deodorizer over the floor of chicken houses. The same statement is made as to the noxious gases such as ammonia and hydrogen sulfide in chicken houses at column 1, lines 26 and 27 of Komakine U.S. patent 3,898,324, issued August 5, 1975, which resulted from a division of the application resulting in patent No. 3,776,188.

French Patent Specification No. 1,485,781 discloses the use of citral bisulphite, paraldehyde and metaldehyde as deodorising and antiseptic reagents.

In accordance with the teaching of French Patent Specification No. 1,354,159, an autoxidisable material is dispensed from an aerosol as fine droplets to present a large surface area to air for autoxidation. In column 1 on page 2, FR 1,354,159 teaches that limonene and lemon oil are suitable autoxidizable materials, which each give rise to odour-counteracting materials via the formation of unstable peroxides.

Conventional gas scrubbers have customarily used an aqueous spray of lye (sodium hydroxide or potassium hydroxide) and chlorine. The sprayed treating liquid has a pH of about 11.

In accordance with one aspect of the present invention, therefore, there is provided a method of treating a naturally occurring oil which contains limonene to produce a material for counteracting the pungency of odorous substances; characterised in that the said naturally occurring oil is mixed with one or more reagents selected from sodium bisulfate, potassium bisulfate, sodium bisulfite, potassium bisulfite, sodium metabisulfite, sulfur dioxide, sodium perchlorate, potassium perchlorate, perchloric acid, sodium perborate, potassium perborate, potassium dichromate, potassium permanganate, sodium nitrate, potassium nitrate, ozone, hydrogen peroxide and aqueous sodium peroxide; and thereafter the mixture is suspended in a vehicle comprising glacial acetic acid and one or more of amyl alcohol, amyl acetate and butyl acetate.

The present invention also comprehends a material for counteracting the pugency of odorous substances produced in accordance with the method of the present invention.

In another aspect of the present invention, there is provided a process of counteracting the pungency of odorous substances, which process comprises treating such odorous substances with a material according to the present invention.

In accordance with the present invention, therefore, we treat odorous materials such as those containing anmoniacal substances to counteract their pungency by utilization of oil containing limonene as a natural component, or the aldehyde content of citrus oil which has been oxidized or otherwise treated to provide or activate aldehyde, and suspended in glacial acetic acid and amyl alcohol, amyl acetate or butyl acetate.

The treatment is economical and effective for ammoniacal substances in various forms such as embodied in urine and manure of livestock and poultry or when air-carried or present in wash water. The treating material can be dispensed in various ways by providing it in liquid form which can be sprayed into airstreams of conventional gas scrubbers, or onto floors or litter, or which can be mixed with wash water before or after it is used.

The process of the present invention for counteracting the pungency of ammoniacal substances utilizes principally oil containing limonene as a natural component, or the aldehyde content of processed citrus oil, as the treating material. Citrus oil is in the skin of fruit of the citrus family, including oranges, lemons, limes, tangerines and grapefruit. Citrus oil is high in terpenes, especially d -limonene which is a liquid terpene hydrocarbon C₁₀H₁₆ and it is preferred that the major portion of the oil by weight be obtained from citrus oil. Over 90 percent of orange oil by weight is d -limonene. Celery-seed oil also contains d -limonene as a natural component. Citrus oil contains 2 to 5 percent by weight of the aldehyde citral having the formula C₉H₁₅CHO.

In preparing the pungency-counteracting treating material according to the present invention, the naturally occurring oil containing limonene as a natural component is processed in the presence of one or more of the said reagents, such as by being oxygenated, which may convert a greater or lesser amount of the limonene to substances which act as deodorizers.

Limonene and the said deodorizing substances derived from limonene may be substantially insoluble in water. Since the processed citrus oil is not water soluble, it is difficult to dispense it effectively for treating ammoniacal substances whether such substances are air-carried or are present in wash water, for example. Consequently, the treating material is supplied in the form of a suspension in glacial acetic acid and amyl alcohol, amyl acetate and/or butyl acetate.

Initially, the treating material may be supplied in somewhat concentrated form and subsequently extended. In preparing the concentrated form, the citrus oil or oil containing limonene as a natural component is processed, such as being oxygenated. A preferred composition for the concentrate is:

| | Weight |
|---|---|
| citrus oil or oil containing limonene as a natural component | 70.5 % |
| glacial acetic acid | 20.0 % |
| amyl acetate, butyl acetate or amyl alcohol | 5.0 % |
| concentrated sulfuric acid | 1.5 % |
| diluted sulfuric acid (1 part acid to 4 parts water by volume) | 2.0 % |
| oxidizing or treating agent (sodium or potassium bisulfate, sodium or potassium bisulfite, sodium metabisulfite, sulfur dioxide, sodium or potassium perchlorate, perchloric acid, sodium or potassium perborate, potassium dichromate, potassium permanganate, sodium or potassium nitrate, ozone, hydrogen peroxide and/or aqueous sodium peroxide) | 1.0 % |
| Total | 1̅0̅0̅.̅0̅ %̅ |

It is preferred that amyl alcohol, rather than amyl acetate or butyl acetate, be used, but either material is suitable.

The 2% diluted sulfuric acid reacts with or catalyses the action of the treating agent. The amount of treating agent could vary from 0.1% to 2% and the amount of diluted sulfuric acid should be altered correspondingly.

If ozone is to be used as an oxidizing or treating agent it could be supplied by an ozone generator such as ultravoilet light.

The various oxygenting substances may be supplied on the basis of the oyxgen made available, not equal weight. Potassium dichromate is the preferred treating agent.

Other suitable aldehydes such as vanillin CH₃₀(OH)C₆H₃CHO, which may be an unrefined form obtained from lignin material, syringaldehyde (CH₃O)₂(OH)C₆H₂CHO, anisaldehyde CH₃OC₆H₄CHO or benzaldehyde C₆H₅CHO may be added to the said treating material. Typically up to 10 percent by weight, preferably 1 percent to 2 percent, of aldehyde based on the amount of the naturally occurring oil may be added.

The treating material may further include a metal selected from copper, aluminium, iron, nickel, zinc, silver, potassium and tin. Preferably, the treating material includes a copper compound, typically, copper sulphate pentahydrate CuSO₄.5H₂O.

In preparing the concentrate, the oil may be processed by adding the treating agent, such as potassium dichromate, to it and mixing. To the mixture the diluted sulfuric acid may be added slowly, such as drop by drop, while the mixing is continued for approximately 15 minutes. The mixture then is allowed to remain undisturbed for a considerable period of time, such as four hours or more. During such period, a green precipiate forms and the material is washed with water successively and decanted until the green material has all been removed in the wash water and the remaining liquid is clear.

Then the suspending agent amyl alcohol, amyl acetate or butyl acetate and the glacial acetic acid is added to the processed oil mixture and the combination is thoroughly mixed with the concentrated sulfuric acid after which the mixture is allowed to stand. After settling is complete the liquid is decanted, any material settling out being discarded.

The concentrate thus prepared may be diluted for use as treating material for counteracting the pungency of ammoniacal substances according to the following formula:

| | Weight |
|---|---|
| concentrate | 65.9 % |
| amyl alcohol, amyl acetate or butyl acetate | 17.0 % |
| glacial acetic acid | 17.0 % |
| copper sulfate pentahydrate | 0.1 % |

Whatever copper content can be incorporated in the treating material provides beneficial deodorizing capabilities, but it is difficult to incorporate metal content in any form. The copper sulfate pentahydrate may be incorporated in the treating material up to the amount indicated. Alternatively or additionally, inclusion of any amount of aluminum, iron, nickel, zinc, silver, potassium or tin is also desirable.

The resulting treating material is not water soluble, but the various ingredients are maintained in substantially homogeneous suspension in the glacial acetic acid and amyl alcohol, amyl acetate or butyl acetate. Amyl acetate or butyl acetate may serve as an emulsifier to promote miscibility of the oil with water and the glacial acetic acid also serves as a carrier to maintain the oil in suspension in a water environment. Consequently, the treating material is suitable for application by spray into a moist gas scrubber which may be of conventional type, or onto a floor or litter, for example, or may be mixed with wash water. The major portion of such treating material by weight is or results from the citrus oil or oil containing limonene as a natural component. The amount of treating material used should be from 5 to 100 parts per million of the material being treated by weight.

The glacial acetic acid increases the acidity of the treating material to some extent, but the sulfuric acid considerably increases the acidity. The high acidity preserves the homogeneity of the material. Also, various types of aldehyde material, including processed citrus oil, vanillin, anisaldehyde and benzaldehyde, are much more effective as deodorants, or to counteract pungency such as produced by ammoniacal substances, if they are in an acid environment.

In treating gas in a scrubber, for example, the treating material may be dispensed to the gas stream in water that may be pumped from a reservoir of perhaps 18900 litre (5,000 gallons) capacity, recovered and returned to the reservoir. Treating material is replenished progressively or periodically, such as at the rate of 17 litre (4 ½ gallons) per day. The pH balance of 6.4 to 7.5 of the sprayed liquid may be maintained by adding concentrated sulfuric acid to the reservoir at the rate of about 17 litre (4 ½ gallons) per day. In some instances it will be desirable for the aqueous treating liquid to have a pH within the range of pH 1 to pH 4, or in some cases even less than pH 1. Such pH range can be obtained by acidifying the aldehyde material such as with sulfuric acid.

If the treating material is to be sprayed into a gas stream, such as in a conventional gas scrubber, it may be desirable to spray simultaneously into the gas stream a wetting agent such as a sulfonated alcohol to improve the contact between the treating material and the air.

Instead of treating wash water with the treating material after the wash water has been used for washing, the treating material can be mixed with water prior to it being used for washing equipment in a cannery or food processing plant, for example, so as to control creation of pungency by the wash water during use.

## Claims

1. A method of treating a naturally occurring oil which contains limonene to produce a material for counteracting the pungency of odorous substances; characterised in that the said naturally occurring oil is mixed with one or more reagents selected from sodium bisulfate, potassium bisulfate, sodium bisulfite, potassium bisulfite, sodium metabisulfite, sulfur dioxide, sodium perchlorate, potassium perchlorate, perchloric acid, sodium perborate, potassium perborate, potassium dichromate, potassium permanganate, sodium nitrate, potassium nitrate, ozone, hydrogen peroxide and aqueous sodium peroxide; and thereafter the mixture is suspended in a vehicle comprising glacial acetic acid and one or more of amyl alcohol, amyl acetate and butyl acetate.

2. The method as claimed in claim 1 characterised in that the naturally occurring oil includes a citrus oil produced from the skin of a fruit of the citrus family.

3. The method as claimed in claim 1 or claim 2, characterised in that the naturally occurring oil is acidified.

4. The method as claimed in any preceding claim, characterised in that the naturally occurring oil is acidified with sulfuric acid.

5. A treating material for counteracting the pungency of odorous substances, which treating material has been produced by a method as claimed in any preceding claim.

6. A treating material as claimed in claim 5, characterised in that the treating material further includes a metal selected from copper, aluminium, iron, nickel, zinc, silver, potassium and tin.

7. A treating material as claimed in claim 5 or claim 6, characterised in that the treating material further includes a copper compound.

8. A treating material as claimed in claim 5, claim 6 or claim 7, characterised in that the treating material further includes copper sulfate pentahydrate.

9. A treating material as claimed in any of claims 5 to 8, characterised in that the naturally occurring oil is mixed with a liquid containing aldehyde.

10. A treating material as claimed in claim 9, characterised in that the aldehyde is selected from vanillin, syringaldehyde, anisaldehyde and benzaldehyde.

11. A process of counteracting the pungency of odorous substances, which process comprises treating such odorous substances with a treating material as claimed in any of claims 5 to 10.

## Patentansprüche

1. Verfahren zur Behandlung eines natürlich vorkommenden, Limonen enthaltenden Öls zur Erzeugung eines Materials, das der Schärfe im Geruch von riechenden Substanzen entgegenwirkt,
dadurch gekennzeichnet, daß das natürlich vorkommende Öl mit einem oder mehreren Reagentien gemischt wird, die aus der Gruppe Natriumbisulfat, Kaliumbisulfat, Natriumbisulfit, Kaliumbisulfit, Natriummetabisulfit, Schwefeldioxid, Natriumperchlorat, Kaliumperchlorat, Perchlorsäure, Natriumperborat, Kaliumperborat, Kaliumdichromat, Kaliumpermanganat, Natriumnitrat, Kaliumnitrat, Ozon, Wasserstoffperoxid und Natriumperoxid in Wasser ausgewählt sind, und danach die Mischung in einem Träger suspendiert wird, der Eisessig und eine oder mehrere der Verbindungen Amylalkohol, Amylacetat und Butylacetat enthält.

2. Verfahren nach Anspruch 1, dadurch gekennzeichnet, daß das natürlich vorkommende Öl ein Citrusöl enthält, das aus der Schale einer Frucht der Citrus-Gattung gewonnen wurde.

3. Verfahren nach Anspruch 1 oder 2, dadurch gekennzeichnet, daß das natürlich vorkommende Öl angesäuert wird.

4. Verfahren nach einem der vorstehenden Ansprüche, dadurch gekennzeichnet, daß das natürlich vorkommende Öl mit Schwefelsäure angesäuert wird.

5. Behandlungsmaterial als Gegenmittel gegen Schärfe im Geruch von riechenden Substanzen, das durch ein Verfahren nach einem der vorstehenden Ansprüche hergestellt worden ist.

6. Behandlungsmaterial nach Anspruch 5, dadurch gekennzeichnet, daß das Behandlungsmaterial weiterhin ein aus Kupfer, Aluminium, Eisen, Nickel, Zink, Silber, Kalium und Zinn ausgewähltes Metall enthält.

7. Behandlungsmaterial nach Anspruch 5 oder Anspruch 6, dadurch gekennzeichnet, daß das Behandlungsmaterial weiterhin eine Kupferverbindung enthält.

8. Behandlungsmaterial nach Anspruch 5, Anspruch 6 oder Anspruch 7, dadurch gekennzeichnet, daß das Behandlungsmaterial weiterhin Kupfersulfatpentahydrat enthält.

9. Behandlungsmaterial nach einem der Ansprüche 5 bis 8, dadurch gekennzeichnet, daß das natürlich vorkommende Öl mit einer aldehydhaltigen Flüssigkeit vermischt ist.

10. Behandlungsmaterial nach Anspruch 9, dadurch gekennzeichnet, daß der Aldehyd aus Vanillin, Syringaaldehyd, Anisaldehyd und Benzaldehyd ausgewählt ist.

11. Verfahren zur Gegenwirkung gegen die Schärfe im Geruch von riechenden Substanzen, bei dem solche riechenden Substanzen mit einem Behandlungsmaterial nach einem der Ansprüche 5 bis 10 behandelt werden.

## Revendications

1. Un procédé de traitement d'une essence naturelle qui contient du limonène pour produire un matériau pour combattre l'odeur irritante de substances odorantes ; caractérisé en ce que ladite huile naturelle est mélangée avec un ou plusieurs réactifs choisis parmi le bisulfate de sodium, le bisulfate de potassium, le bisulfite de sodium, le bisulfite de potassium, le métabisulfite de sodium, le dioxyde de soufre, le perchlorate de sodium, le perchlorate de potassium, l'acide perchlorique, le perborate de sodium, le perborate de potassium, le dichromate de potassium, le permanganate de potassium, le nitrate de sodium, le nitrate de potassium, l'ozone, le peroxyde d'hydrogène et le peroxyde de sodium aqueux ; et qu'ensuite, le mélange est mis en suspension dans un véhicule comprenant de l'acide acétique glacial et un ou plusieurs alcools amyliques, de l'acétate d'amyle et de l'acétate de butyle.

2. Le procédé selon la revendication l caractérisé en ce que l'essence naturelle inclut une essence de citrus produite à partir de la peau d'un fruit de la famille des *Citrus*.

3. Le procédé selon la revendication 1 ou la revendication 2, caractérisé en ce que l'essence naturelle est acidifiée.

4. Le procédé selon l'une quelconque des revendications préécdentes, caractérisé en ce que l'essence naturelle est acidifiée avec de l'acide sulfurique.

5. Un produit traitant pour combattre l'odeur irritante de substances odorantes, lequel produit traitant a été produit par un procédé selon l'une quelconque des revendications précédentes.

6. Un produit traitant selon la revendication 5, caractérisé en ce que le produit traitant inclut de plus un métal choisi parmi le cuivre, l'aluminium, le fer, le nickel, le zinc , l'argent, le potassium et l'étain.

7. Un produit traitant selon la revendication 5 ou la revendication 6, caractérisé en ce que le produit traitant inclut de plus un composé contenant du cuivre.

8. Un produit traitant selon la revendication 5, la revendication 6 ou la revendication 7, caractérisé en ce que le produit traitant inclut de plus du sulfate de cuivre pentahydraté.

9. Un produit traitant selon l'une quelconque des revendications 5 à 8, caractérisé en ce que l'essence naturelle est mélangée avec un liquide contenant un aldéhyde.

10. Un produit traitant selon la revendication 9, caractérisé en ce que l'aldéhyde est choisi parmi la vanilline, le syringaldéhyde, l'anisaldéhyde et le benzaldéhyde.

11. Un traitement de lutte contre l'odeur irritante de substances odorantes, lequel procédé comprend de traiter ces substances odorantes avec un produit traitant selon l'une quelconque des revendications 5 à 10.
